(19) Europäisches Patentamt
European Patent Office
Office européen des brevets

(11) **EP 3 058 056 B1**

(12) **EUROPÄISCHE PATENTSCHRIFT**

(45) Veröffentlichungstag und Bekanntmachung des Hinweises auf die Patenterteilung:
**09.09.2020  Patentblatt 2020/37**

(21) Anmeldenummer: **14781571.6**

(22) Anmeldetag: **09.10.2014**

(51) Int Cl.:
*C11D 3/386* *(2006.01)*    *C11D 3/37* *(2006.01)*

(86) Internationale Anmeldenummer:
**PCT/EP2014/071611**

(87) Internationale Veröffentlichungsnummer:
**WO 2015/055491 (23.04.2015 Gazette 2015/16)**

(54) **STABILISIERUNG VON ENZYMEN IN TENSIDHALTIGEN WÄSSRIGEN SYSTEMEN**

STABILIZATION OF ENZYMES IN DETERGENT-CONTAINING AQUEOUS SYSTEMS

STABILISATION D'ENZYMES DANS DES SYSTÈMES AQUEUX CONTENANT DES DÉTERGENTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.10.2013  DE 102013017047**

(43) Veröffentlichungstag der Anmeldung:
**24.08.2016  Patentblatt 2016/34**

(73) Patentinhaber:
• **Henkel AG & Co. KGaA**
**40589 Düsseldorf (DE)**
• **Fraunhofer-Gesellschaft zur Förderung der angewandten Forschung e.V.**
**80686 München (DE)**

(72) Erfinder:
• **HELLMUTH, Hendrik**
**40237 Düsseldorf (DE)**
• **BODE, Nicole**
**40212 Düsseldorf (DE)**
• **O'CONNELL, Timothy**
**40547 Düsseldorf (DE)**
• **WEBER, Thomas**
**41541 Dormagen (DE)**
• **LAUFS, Brian**
**41363 Jüchen (DE)**
• **VOCKENROTH, Inga Kerstin**
**40597 Düsseldorf (DE)**
• **LASCHEWSKY, André**
**14469 Potsdam (DE)**
• **WISCHERHOFF, Erik**
**14469 Potsdam (DE)**
• **PÄCH, Michael**
**14471 Potsdam (DE)**

(56) Entgegenhaltungen:
**WO-A1-00/70006        WO-A1-2011/001173
WO-A1-2013/034438**

**Beschreibung**

Die vorliegende Erfindung betrifft die Verwendung bestimmter Polymere zur Verstärkung oder nach Lagerung zumindest zum Erhalt der Leistung von Enzymen in wasser- und tensidhaltigen Wasch- oder Reinigungsmitteln, die beim Waschen von Textilien oder Reinigen harter Oberflächen zum Einsatz kommen.

Zur Erhöhung ihrer Wasch- oder Reinigungsleistung enthalten Wasch- oder Reinigungsmittel häufig mindestens ein Enzym, oft mehrere Enzyme. Häufig eingesetzte Enzyme sind dabei Proteasen, Lipasen, Amylasen, Cellulasen und Mannanasen.

Insbesondere in flüssigen Wasch- und Reinigungsmitteln, in denen die enzymatischen Wirkstoffe in gelöster oder suspendierter Form vorliegen, kann es durch den direkten Kontakt der Enzyme mit den weiteren Inhaltsstoffen und/oder untereinander zu unerwünschten Wechselwirkungen kommen, welche die Lagerstabilität der Enzyme in der flüssigen Formulierung negativ beeinflussen. So ist zum Beispiel bekannt, dass proteolytische Enzyme andere in einem flüssigen Wasch- oder Reinigungsmittel enthaltene Enzyme, wie Amylasen, Lipasen oder Cellulasen, aber auch in besonders ungünstigen Fällen sich selbst, abbauen. Auch wenn die Enzyme nicht durch Abbau in ihrer Primärstruktur zerstört werden, kann es durch Einflüsse sonstiger Wasch- und Reinigungsmittelinhaltsstoffe zu Veränderungen in der Faltung der Tertiärstruktur von Enzymen kommen. Als Resultat wird mit zunehmender Lagerungsdauer ein über die Zeit zunehmender Verlust der Reinigungsleistung des Mittels gegenüber Enzym-spezifischen Anschmutzungen beobachtet. Zu den Wasch- und Reinigungsmittelinhaltsstoffen, welche in wässrigen Systemen zur Inaktivierung von Enzymen führen können, zählen synthetische anionische Tenside, insbesondere solche vom Alkylbenzolsulfonat-Typ.

Der Einsatz von Poly-(N-vinylpyrrolidon) in Waschmitteln ist bekannt. So beschreibt zum Beispiel die internationale Patentanmeldung WO 2011/001173 A1 Flüssigwaschmittel, die 0,01 bis 5 Gew.-% Cellulase und 0,01 bis 5 Gew.-% Poly-(N-vinylpyrrolidon) und/oder dessen Salz mit einem mittleren Molekulargewicht von 20000 g/mol bis 60000 g/mol enthalten. Aus der internationalen Patentanmeldung WO 97/29139 A1 sind vernetzte Polymere aus 10 bis 50 Gew.-% N-Vinylcaprolactam und 50 bis 90 Gew.-% N-Vinylpyrrolidon bekannt, die in Gegenwart von 0,5 bis 7 Gew.-% eines Vernetzers, der auch in situ erzeugtes 1-Vinyl-3(E)-ethylidenpyrrolidon sein kann, hergestellt werden können. Derartige vernetzte Polymere sind zum Herausfiltern von Polyphenolen aus Bier geeignet. Die soil-release-Wirkung von N-Vinylcaprolactam-Homopolymeren und -Copolymeren mit untergeordneten Mengen an anderen Monomeren wie beispielsweise N-Vinylpyrrolidon ist aus der europäischen Patentanmeldung EP 0 181 204 A2 bekannt. Aus der europäischen Patentanmeldung EP 0 181 205 A2 ist bekannt, dass solche Polymere zum Erzielen des soil-release-Effekts auch als Umhüllungsmaterialien auf Fasern, insbesondere aus Polyester, aufgebracht werden können. Aus der US-amerikanischen Patentanmeldung US 2002/0177542 sind Waschmittel bekannt, die eine soil-relase-Effekt aufweisende und gewebeweichmachende Menge an N-Vinylcaprolactam-Homopolymer mit einem K-Wert von mindestens 40 enthalten. Die internationale Patentanmeldung WO 2004/014326 A1 beschreibt aniontensidhaltige Haarwaschmittel, die Amino- und Hydroxygruppen aufweisende Silikonderivate und wasserlösliche kationische Polymere mit mittlerem Molekulargewicht von 100000 g/mol bis 2000000 g/mol und Ladungsdichten von 0,6 bis 4 meq/g enthalten, wobei unter diesen auch N-vinylpyrrolidon/Alkylaminoacrylat/N-Vinylcaprolactam-Copolymere genannt werden, und die dort wegen ihres konditionierenden Effekts eingesetzt werden. Aus der internationalen Patentanmeldung WO 2013/034438 A1 ist bekannt, dass durch Polymerisation von N-Vinylcaprolactam, N-Vinylpyrrolidon, N-Vinylpiperidon, N-Vinylsuccinimid, N-Vinylglutarimid, N-Vinylacetamid, N-Alkyl-N-Vinylacetamid, N-Vinylformamid, N-Alkyl-N-Vinylformamid und/oder Mischungen aus mindestens 2 dieser Monomeren zugängliche Polymere die Primärwaschkraft von Wasch- oder Reinigungsmitteln beim Waschen von Textilien oder beim Reinigen harter Oberflächen gegenüber insbesondere bleich- oder enzymsensitiven Anschmutzungen verstärken. Aus der internationalen Patentanmeldung WO 00/70006 ist bekannt, dass die Kombination aus Enzym und Acrylsäure-basiertem Polymer zu einer besseren Fleckentfernung des Mittels führt.

Überraschenderweise wurde gefunden, dass bestimmte N-Vinylpyrrolidon-haltige Polymere zur Lösung der oben angesprochenen Aufgabe der Verbesserung der Enzymstabilität beitragen. Gegenstand der Erfindung ist die Verwendung von durch Polymerisation von N-Vinylpyrrolidon mit einem Co-Monomeren, ausgewählt aus der Gruppe umfassend N-Vinylcaprolactam, N-Vinylpiperidon, N-Vinylsuccinimid, N-Vinylglutarimid, N-Vinylacetamid, N-Alkyl-N-Vinylacetamid, N-Vinylformamid, N-Alkyl-N-Vinylformamid, Dialkylacrylamid und Mischungen aus mindestens 2 von diesen, erhältlichen Polymeren

- zur Stabilisierung von Enzymen und/oder
- zur Verlangsamung des Enzymabbaus und/oder
- zur Verhinderung des Enzymabbaus

in flüssigen wasser- und tensidhaltigen Wasch- oder Reinigungsmitteln.

Eine Alkylgruppe in den genannten Monomeren weist vorzugsweise 1 bis 10 C-Atome, insbesondere 1 bis 3 C-Atome auf und kann linear oder ein- oder gegebenenfalls mehrfach verzweigt sein; besonders bevorzugt ist die Methylgruppe. Die erfindungsgemäß eingesetzten Polymere sind vorzugsweise nicht vernetzt. Das Polymer ist vorzugsweise aus neben aus dem Monomer N-Vinylpyrrolidon stammenden Einheiten aus 1 weiterem Monomer stammenden Einheiten zusammengesetzt, und weist die beiden genannten Einheiten im Gewichtsverhältnis von 99:1 bis 1:99, insbesondere von 97:3 bis 70:30, auf.

Der polymere Wirkstoff weist vorzugsweise ein mittleres Molekulargewicht (hier und im Folgenden bei mittleren Molekulargewichtsangaben: Zahlenmittel) im Bereich von 1000 g/mol bis 500000 g/mol, insbesondere von 1100 g/mol bis 150000 g/mol auf.

Durch die Erfindung gelingt es, ein enzym-, tensid- und wasserhaltiges flüssiges Wasch- oder Reinigungsmittel bereitzustellen, welches dauerhaft eine verbesserte Wasch- oder Reinigungsleistung, insbesondere bei längerer Lagerung, aufweist.

Vorzugsweise zeigt das Polymer Wechselwirkungen mit anionischen Tensiden wie insbesondere linearem Alkylbenzolsulfonat, die auf die Ausbildung eines Tensid-Polymer-Aggregats zurückzuführen sein können. Der Effekt kann über eine Messung der Oberflächen- oder Grenzflächenspannung nachgewiesen werden, wobei die Oberflächen- oder Grenzflächenspannung durch die Anwesenheit des Polymers erhöht wird. Diese Erhöhung kann darauf beruhen, dass sich ein reinigungsaktives Aggregat in der Lösung bildet und daher weniger Tensid an der Grenzfläche vorhanden ist. Zur Bestimmung des Aggregationsparameters $X_{ag}$ wird die Oberflächenspannung $\gamma$ einer wässrigen Lösung von 0,12 g/l linearem $C_{10-13}$-Alkylbenzolsulfonat, wie es zum Beispiel unter den Handelsnamen Disponil® LDBS 55 oder Marlon® A360 erhältlich ist, in Ab- und Anwesenheit von 0,2 g/l des Polymers gemessen und der Wert in Abwesenheit des Polymers von dem Wert in Anwesenheit des Polymers subtrahiert:

$$X_{ag} = \gamma_1 \,(\text{Tensid + Polymer}) - \gamma_2 \,(\text{Tensid})$$

Dabei kann die Messung der Oberflächenspannung mittels der Du-Noüy Ringmethode, beispielsweise unter Einsatz eines TE3-Ring/Platten Tensiometers der Firma Lauda (Lauda-Königshofen) vorgenommen werden. Hierzu wird ein Ring aus beispielsweise Metall, der an einem Torsionskraftmesser befestigt ist, so in die Tensid-Polymer-Lösung eingetaucht, dass sich der Ring unter der Oberfläche der Lösung befindet. Der Ring wird dann langsam aus der Lösung gezogen und die Kraft, die auf den Messring ausgeübt wird, kurz bevor der Flüssigkeitsfilm abreißt, wird mit dem Torsionskraftmesser gemessen. Die Oberflächenspannung kann bei Kenntnis des Durchmessers des Ringes und der Abreißkraft berechnet werden.

Alternativ kann der Aggregationsparameters $X_{ag}$ durch die Messung der dynamischen Grenzflächenspannung, beispielsweise mittels Tropfen-Volumen-Tensiometrie, die zum Beispiel mit Hilfe eines TVT2-Tropfen-Volumen-Tensiometers der Firma Lauda (Lauda-Königshofen) durchgeführt werden kann, bestimmt werden. Hierzu wird Isopropylmyristat aus einer Kanüle in die wässrige Lösung von 0,12 g/l des oben genannten linearen $C_{10-13}$-Alkylbenzolsulfonats in Ab- und Anwesenheit von 0,2 g/l des Polymers gedrückt. Die Messung des Tropfenvolumens erlaubt die Berechnung der dynamischen Grenzflächenspannung, wobei das Tropfenvolumen nach 1 Minute zu messen ist. Der Wert in Abwesenheit des Polymers wird von dem Wert in Anwesenheit des Polymers subtrahiert und das Resultat mit einem Normierungsfaktor 3 multipliziert:

$$X_{ag} = 3 \text{ x } [\gamma_{1\text{Grenzfläche}} \,(\text{Tensid + Polymer}) - \gamma_{2\text{Grenzfläche}} \,(\text{Tensid})]$$

Die Messungen werden jeweils bei 25°C unter Einstellung der Messlösungen auf pH 8,5 vorgenommen. Besonders bevorzugt sind Polymere, bei denen Aggregationsparameter $X_{ag} > 1$ mN/m, vorzugsweise $X_{ag} > 4$ mN/m und insbesondere im Bereich von 5 mN/m bis 8 mN/m auftreten.

Die erfindungsgemäß verwendeten Wirkstoffe sind auf einfachem Wege durch radikalische Polymerisation der ethylenisch ungesättigten mindestens 2 Monomere herstellbar, wobei sie vorzugsweise als statistische Copolymerisation durchgeführt wird. Gewünschtenfalls können in den Polymeren jedoch auch Blöcke aus Vinylpyrrolidon-Einheiten und Einheiten aus einem Co-Monomer und gegebenenfalls weitere Blöcke aus weiteren Co-Monomeren vorliegen.

Der erfindungsgemäß erreichte enzmstabilisierende Effekt kann sich im Rahmen eines Wasch- oder Reinigungsprozesses bemerkbar machen, wenn man den polymeren Wirkstoff als Bestandteil eines Wasch- oder Reinigungsmittels in die Flotte einbringt, wobei die Konzentration an polymerem Wirkstoff in der Flotte vorzugsweise im Bereich von 0,01 g/l bis 0,5 g/l, insbesondere von 0,02 g/l bis 0,2 g/l liegt.

Der erfindungsgemäß erreichte enzmstabilisierende Effekt tritt bei Einsatz eines Enzyms, aber auch mehrerer Enzyme auf. Prinzipiell sind diesbezüglich alle im Stand der Technik etablierten Enzyme einsetzbar. Vorzugsweise handelt es sich um eines oder mehrere Enzyme, die in einem Wasch- oder Reinigungsmittel eine katalytische Aktivität entfalten können, insbesondere eine Protease, Amylase, Lipase, Cellulase, Hemicellulase, Mannanase, ein Pektinspaltendes Enzym, eine Tannase, Xylanase, Xanthanase, β-Glucosidase, Carrageenase, Perhydrolase, Oxidase, Oxidoreduktase, sowie um deren Gemische. Bevorzugte hydrolytische Enzyme umfassen insbesondere Proteasen, Amylasen, insbesondere α-Amylasen, Cellulasen, Lipasen, Hemicellulasen, insbesondere Pectinasen, Mannanasen, β-Glucanasen, sowie deren Gemische. Besonders bevorzugt sind Proteasen, Amylasen und/oder Lipasen sowie deren Gemische und ganz besonders bevorzugt sind Lipasen. Diese Enzyme sind im Prinzip natürlichen Ursprungs; ausgehend von den natürlichen Molekülen stehen für den Einsatz in Wasch- oder Reinigungsmitteln verbesserte Varianten zur Verfügung, die entsprechend bevorzugt eingesetzt werden.

Im Folgenden werden Beispiele für die verschiedenen Enzymklassen, die im Rahmen der vorliegenden Erfindung eingesetzt werden können, beschrieben:

Beispiele für verwendbare Lipasen (Triacylglycerolacylhydrolasen, EC 3.1.1.3) oder Cutinasen, die insbesondere wegen ihrer Triglycerid-spaltenden Aktivitäten enthalten sind, aber auch, um aus geeigneten Vorstufen in situ Persäuren zu erzeugen, sind die ursprünglich aus Humicola lanuginosa (Thermomyces lanuginosus) erhältlichen oder die entsprechenden weiterentwickelten Lipasen, insbesondere solche mit dem Aminosäureaustausch D96L. Sie werden beispielsweise von der Firma Novozymes unter den Handelsnamen Lipolase®, Lipolase®Ultra, LipoPrime®, Lipozyme® und Lipex® vertrieben. Des Weiteren sind beispielsweise die Cutinasen einsetzbar, die ursprünglich aus Fusarium solani pisi und Humicola insolens isoliert worden sind. Ebenso brauchbare Lipasen sind von der Firma Amano unter den Bezeichnungen Lipase CE®, Lipase P®, Lipase B®, beziehungsweise Lipase CES®, Lipase AKG®, Bacillus sp. Lipase®, Lipase AP®, Lipase M-AP® und Lipase AML® erhältlich. Von der Firma Genencor sind beispielsweise die Lipasen beziehungsweise Cutinasen einsetzbar, deren Ausgangsenzyme ursprünglich aus Pseudomonas mendocina und Fusarium solanii isoliert worden sind. Als weitere wichtige Handelsprodukte sind die ursprünglich von der Firma Gist-Brocades vertriebenen Präparationen M1 Lipase® und Lipomax® und die von der Firma Meito Sangyo KK, Japan, unter den Namen Lipase MY-30®, Lipase OF® und Lipase PL® vertriebenen Enzyme zu erwähnen, ferner das Produkt Lumafast® von der Firma Genencor. In verschiedenen bevorzugten Ausführungsformen werden Lipasen aus den Familie I.1 oder I.2 von Lipasen (gemäß Apigny & Jaeger, Biochem. J. (1999) 343, 177-183), insbesondere eine Lipase aus einem Bakterium der Gattung Burkholeria oder Pseudomonas verwendet. Ein spezielles Beispiel für eine geeignete Lipase ist die Lipase lipA aus Burkholderia cepacia.

Unter den Proteasen sind solche vom Subtilisin-Typ bevorzugt. Beispiele hierfür sind die Subtilisine BPN' und Carlsberg, die Protease PB92, die Subtilisine 147 und 309, die Alkalische Protease aus Bacillus lentus, Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase® von der Firma Novozymes A/S, Bagsvaerd, Dänemark, erhältlich. Die Subtilisine 147 und 309 werden unter den Handelsnamen Esperase®, beziehungsweise Savinase® von der Firma Novozymes vertrieben. Von der Protease aus Bacillus lentus DSM 5483 leiten sich die unter der Bezeichnung BLAP® geführten Protease-Varianten ab. Weitere brauchbare Proteasen sind beispielsweise die unter den Handelsnamen Durazym®, Relase®, Everlase®, Nafizym®, Natalase®, Kannase® und Ovozyme® von der Firma Novozymes, die unter den Handelsnamen, Purafect®, Purafect® OxP, Purafect® Prime, Excellase® und Properase® von der Firma Genencor, das unter dem Handelsnamen Protosol® von der Firma Advanced Biochemicals Ltd., Thane, Indien, das unter dem Handelsnamen Wuxi® von der Firma Wuxi Snyder Bioproducts Ltd., China, die unter den Handelsnamen Proleather® und Protease P® von der Firma Amano Pharmaceuticals Ltd., Nagoya, Japan, und das unter der Bezeichnung Proteinase K-16 von der Firma Kao Corp., Tokyo, Japan, erhältlichen Enzyme. Besonders bevorzugt eingesetzt werden auch die Proteasen aus Bacillus gibsonii und Bacillus pumilus.

Beispiele für verwendbare Amylasen sind die α-Amylasen aus Bacillus licheniformis, aus B. amyloliquefaciens oder aus B. stearothermophilus sowie deren für den Einsatz in Wasch- oder Reinigungsmitteln verbesserte Weiterentwicklungen. Das Enzym aus B. licheniformis ist von der Firma Novozymes unter dem Namen Termamyl® und von der Firma Genencor unter dem Namen Purastar®ST erhältlich. Weiterentwicklungsprodukte dieser α-Amylase sind von der Firma Novozymes unter den Handelsnamen Duramyl® und Termamyl®ultra, von der Firma Genencor unter dem Namen Purastar®OxAm und von der Firma Daiwa Seiko Inc., Tokyo, Japan, als Keistase® erhältlich. Die α-Amylase von B. amyloliquefaciens wird von der Firma Novozymes unter dem Namen BAN® vertrieben, und abgeleitete Varianten von der α- Amylase aus B. stearothermophilus unter den Namen BSG® und Novamyl®, ebenfalls von der Firma Novozymes. Des Weiteren sind für diesen Zweck die α-Amylase aus Bacillus sp. A 7-7 (DSM 12368) und die Cyclodextrin-Glucanotransferase (CGTase) aus B. agaradherens (DSM 9948) hervorzuheben. Ebenso sind Fusionsprodukte aller genannten Moleküle einsetzbar. Darüber hinaus sind die unter den Handelsnamen Fungamyl® von dem Unternehmen Novozymes erhältlichen Weiterentwicklungen der α-Amylase aus Aspergillus niger und A. oryzae geeignet. Weitere vorteilhaft einsetzbare Handelsprodukte sind beispielsweise die Amylase-LT® und Stainzyme® oder Stainzyme ultra® bzw. Stainzyme plus®, letztere ebenfalls von dem Unternehmen Novozymes. Auch durch Punktmutationen erhältliche Varianten dieser Enzyme können erfindungsgemäß eingesetzt werden. Erfindungsgemäß konfektionierbare Amylasen sind ferner vorzugsweise α-Amylasen.

Cellulasen können je nach Zweck als reine Enzyme, als Enzympräparationen oder in Form von Mischungen, in denen sich die einzelnen Komponenten vorteilhafterweise hinsichtlich ihrer verschiedenen Leistungsaspekte ergänzen, vorhanden sein. Zu diesen Leistungsaspekten zählen insbesondere die Beiträge der Cellulase zur Primärwaschleistung des Mittels (Reinigungsleistung), zur Sekundärwaschleistung des Mittels (Antiredepositionswirkung oder Vergrauungsinhibition), zur Avivage (Gewebewirkung) oder zur Ausübung eines "stone washed"-Effekts. Eine brauchbare pilzliche, Endoglucanase(EG)-reiche Cellulase-Präparation, beziehungsweise deren Weiterentwicklungen wird von der Firma Novozymes unter dem Handelsnamen Celluzyme® angeboten. Die ebenfalls von der Firma Novozymes erhältlichen Produkte Endolase® und Carezyme® basieren auf der 50 kD-EG, beziehungsweise der 43 kD-EG aus H. insolens DSM 1800. Weitere einsetzbare Handelsprodukte dieser Firma sind Cellusoft®, Renozyme® und Celluclean®. Weiterhin einsetzbar sind beispielsweise die 20 kD-EG aus Melanocarpus, die von der Firma AB Enzymes, Finnland, unter den Handelsnamen Ecostone® und Biotouch® erhältlich sind. Weitere Handelsprodukte der Firma AB Enzymes sind Econase® und Ecopulp®. Weitere geeignete Cellulasen sind aus Bacillus sp. CBS 670.93 und CBS 669.93, wobei die aus Bacillus sp. CBS 670.93 von der Firma Genencor unter dem Handelsnamen Puradax® erhältlich ist. Weitere Handelsprodukte der Firma Genencor sind "Genencor detergent cellulase L" und IndiAge®Neutra. Auch durch Punktmutationen erhältliche Varianten dieser Enzyme können erfindungsgemäß eingesetzt werden.

Ferner können insbesondere zur Entfernung bestimmter Problemanschmutzungen weitere Enzyme eingesetzt sein, die unter dem Begriff Hemicellulasen zusammengefasst werden. Hierzu gehören beispielsweise Mannanasen, Xanthanlyasen, Xanthanasen, Xyloglucanasen, Xylanasen, Pullulanasen, Pektin-spaltende Enzyme und β-Glucanasen. Die aus Bacillus subtilis gewonnene ß-Glucanase ist unter dem Namen Cereflo® von der Firma Novozymes erhältlich. Erfindungsgemäß besonders bevorzugte Hemicellulasen sind Mannanasen, welche beispielsweise unter den Handelsnamen Mannaway® von dem Unternehmen Novozymes oder Purabrite® von dem Unternehmen Genencor vertrieben werden. Zu den Pektin-spaltenden Enzymen werden im Rahmen der vorliegenden Erfindung ebenfalls Enzyme gezählt mit den Bezeichnungen Pektinase, Pektatlyase, Pektinesterase, Pektindemethoxylase, Pektinmethoxylase, Pektinmethylesterase, Pektase, Pektinmethylesterase, Pektinoesterase, Pektinpektylhydrolase, Pektindepolymerase, Endopolygalacturonase, Pektolase, Pektinhydrolase, Pektin-Polygalacturonase, Endo-Polygalacturonase, Poly-α-1,4-Galacturonid Glycanohydrolase, Endogalacturonase, Endo-D-galacturonase, Galacturan 1,4-α-Galacturonidase, Exopolygalacturonase, Poly(galacturonat) Hydrolase, Exo-D-Galacturonase, Exo-D-Galacturonanase, Exopoly-D-Galacturonase, Exo-poly-α-Galacturonosidase, Exopolygalacturonosidase oder Exopolygalacturanosidase. Beispiele für diesbezüglich geeignete Enzyme sind beispielsweise unter den Namen Gamanase®, Pektinex AR®, X-Pect® oder Pectaway® von dem Unternehmen Novozymes, unter dem Namen Rohapect UF®, Rohapect TPL®, Rohapect PTE100®, Rohapect MPE®, Rohapect MA plus HC, Rohapect DA12L®, Rohapect 10L®, Rohapect B1L® von dem Unternehmen AB Enzymes und unter dem Namen Pyrolase® von dem Unternehmen Diversa Corp., San Diego, CA, USA erhältlich.

Unter all diesen Enzymen sind solche besonders bevorzugt, die an sich gegenüber einer Oxidation vergleichsweise stabil oder beispielsweise über Punktmutagenese stabilisiert worden sind.

Zur Erhöhung der bleichenden Wirkung können in den Wasch- oder Reinigungsmitteln auch Oxidoreduktasen, beispielsweise Oxidasen, Oxygenasen, Katalasen (die bei niedrigen $H_2O_2$-Konzentrationen als Peroxidase reagieren), Peroxidasen, wie Halo-, Chloro-, Bromo-, Lignin-, Glucose- oder Manganperoxidasen, Dioxygenasen oder Laccasen (Phenoloxidasen, Polyphenoloxidasen) enthalten sein. Als geeignete Handelsprodukte sind Denilite® 1 und 2 der Firma Novozymes oder Gluzyme® mono BG der Firma Novozymes zu nennen.

Enzym ist in den Mitteln vorzugsweise in einer Menge von 1 x 10⁻⁸ Gew.-% bis 5 Gew.-% an aktivem Protein, insbesondere 0,001 Gew.-% bis 3 Gew.-%, bevorzugt von 0,01 Gew.-% bis 1,5 Gew.-%, besonders bevorzugt von 0,05 bis 1,25 Gew.-% jeweils bezogen auf das Gesamtgewicht des Wasch- oder Reinigungsmittels, enthalten, wobei auch jedes enthaltene Enzym für sich genommen in den genannten Mengen vorliegen kann, wenn das Mittel mehr als ein Enzym enthält.

Enzymhaltige Wasch- oder Reinigungsmittel, die einen erfindungsgemäß zu verwendenden polymeren Wirkstoff enthalten, weisen Tensid und Wasser auf und können alle üblichen sonstigen Bestandteile derartiger Mittel enthalten, die nicht in unerwünschter Weise mit dem erfindungswesentlichen Wirkstoff wechselwirken. Vorzugsweise wird ein oben definierter polymerer Wirkstoff in Mengen von 0,1 Gew.-% bis 10 Gew.-%, insbesondere von 0,5 Gew.-% bis 2 Gew.-% in Wasch- oder Reinigungsmittel eingearbeitet.

In einer bevorzugten Ausführungsform enthält ein im Rahmen der erfindungsgemäßen Verwendung eingesetztes Mittel synthetisches Aniontensid, insbesondere Alkylbenzolsulfonat, Alkylsulfat, Alkylethersulfat, Alkyl- und/oder Dialkylsulfosuccinat, Sulfofettsäureester und/oder Sulfofettsäuredisalze, insbesondere in einer Menge im Bereich von 0,1 Gew.-% bis 20 Gew.-% und besonders bevorzugt von 2 Gew.-% bis 15 Gew.-%, wobei in ihnen die Alkylgruppe vorzugsweise 8 bis 22, insbesondere 12 bis 18 C-Atome besitzt. Bevorzugt sind die Derivate der Fettalkohole mit insbesondere 12 bis 18 C-Atomen und deren verzweigtkettiger Analoga, der sogenannten Oxoalkohole. Die Alkyl- und Alkenylsulfate können in bekannter Weise durch Reaktion der entsprechenden Alkoholkomponente mit einem üblichen Sulfatierungsreagenz, insbesondere Schwefeltrioxid oder Chlorsulfonsäure, und anschließende Neutralisation mit Alkali-, Ammonium- oder Alkyl- oder Hydroxyalkyl-substituierten Ammoniumbasen hergestellt werden. Zu den einsetzbaren Tensiden vom Sulfat-Typ gehören auch die sulfatierten Alkoxylierungsprodukte der genannten Alkohole, sogenannte Ethersulfate. Vorzugsweise enthalten derartige Ethersulfate 2 bis 30, insbesondere 4 bis 10 Ethylenglykol-Gruppen pro Molekül. Zu den geeigneten Aniontensiden vom Sulfonat-Typ gehören die durch Umsetzung von Fettsäureestern mit Schwefeltrioxid und anschließender Neutralisation erhältlichen $\alpha$-Sulfoester, insbesondere die sich von Fettsäuren mit 8 bis 22 C-Atomen, vorzugsweise 12 bis 18 C-Atomen, und linearen Alkoholen mit 1 bis 6 C-Atomen, vorzugsweise 1 bis 4 C-Atomen, ableitenden Sulfonierungsprodukte, sowie die durch formale Verseifung aus diesen hervorgehenden Sulfofettsäuren. Bevorzugte Aniontenside sind auch die Salze von Sulfobernsteinsäureestern, die auch als Alkylsulfosuccinate oder Dialkylsulfosuccinate bezeichnet werden, und die Monoester oder Diester der Sulfobernsteinsäure mit Alkoholen, vorzugsweise Fettalkoholen und insbesondere ethoxylierten Fettalkoholen darstellen. Bevorzugte Sulfosuccinate enthalten $C_8$- bis $C_{18}$-Fettalkoholreste oder Mischungen aus diesen. Insbesondere bevorzugte Sulfosuccinate enthalten einen ethoxylierten Fettalkoholrest, der für sich betrachtet ein nichtionisches Tenside darstellt. Dabei sind wiederum Sulfosuccinate, deren Fettalkohol-Reste sich von ethoxylierten Fettalkoholen mit eingeengter Homologenverteilung ableiten, besonders bevorzugt. Als Gegenkationen für die synthetischen Aniontenside kommen insbesondere Ionen der Alkalimetalle wie Lithium, Natrium und Kalium, aber auch Ammoniumionen in Betracht, wobei in den Ammoniumionen das N-Atom auch in Summe 1 bis 4 $C_{1-4}$-Alkyl- und/oder $C_{2-4}$-Hydroxyalkylgruppen tragen kann. Bevorzugt wird das synthetische Aniontensid aus den Alkylbenzolsulfonaten ausgewählt, in denen die Alkylgruppe 8 bis 22, insbesondere 10 bis 18 C-Atome besitzt. Bei diesen handelt es sich üblicherweise nicht um Einzelsubstanzen, sondern um Schnitte oder Mischungen. Vorzugsweise wird als alleiniges synthetisches Aniontensid oder als Teil der Gesamtmenge an synthetischem Aniontensid Alkali-$C_{10-13}$-alkylbenzolsulfonat, insbesondere solches, in dem die Alkylreste linear sind, eingesetzt.

Eine weitere Ausführungsform derartiger Mittel umfasst die Anwesenheit von nichtionischem Tensid, ausgewählt aus Fettalkylpolyglykosiden, Fettalkylpolyalkoxylaten, insbesondere -ethoxylaten und/oder -propoxylaten, Fettsäurepolyhydroxyamiden und/oder Ethoxylierungs-und/oder Propoxylierungsprodukten von Fettalkylaminen, vicinalen Diolen, Fettsäurealkylestern und/oder Fettsäureamiden sowie deren Mischungen, insbesondere in einer Menge im Bereich von 2 Gew.-% bis 25 Gew.-%.

Zu den in Frage kommenden nichtionischen Tensiden gehören die Alkoxylate, insbesondere die Ethoxylate und/oder Propoxylate von gesättigten oder ein- bis mehrfach ungesättigten linearen oder verzweigtkettigen Alkoholen mit 10 bis 22 C-Atomen, vorzugsweise 12 bis 18 C-Atomen. Der mittlere Alkoxylierungsgrad der Alkohole liegt dabei in der Regel zwischen 1 und 20, vorzugsweise zwischen 3 und 10. Sie können in bekannter Weise durch Umsetzung der entsprechenden Alkohole mit den entsprechenden Alkylenoxiden hergestellt werden. Geeignet sind insbesondere die Derivate der Fettalkohole, obwohl auch deren verzweigtkettige Isomere, insbesondere sogenannte Oxoalkohole, zur Herstellung verwendbarer Alkoxylate eingesetzt werden können. Brauchbar sind demgemäß die Alkoxylate, insbesondere die Ethoxylate, primärer Alkohole mit linearen, insbesondere Dodecyl-, Tetradecyl-, Hexadecyl- oder Octadecyl-Resten sowie deren Gemische. Außerdem sind entsprechende Alkoxylierungsprodukte von Alkylaminen, vicinalen Diolen und Carbonsäureamiden, die hinsichtlich des Alkylteils den genannten Alkoholen entsprechen, verwendbar. Darüber hinaus kommen die Ethylenoxid- und/oder Propylenoxid-Insertionsprodukte von Fettsäurealkylestern sowie Fettsäurepolyhydroxyamide in Betracht. Zur Einarbeitung in die Mittel geeignete sogenannte Alkylpolyglykoside sind Verbindungen der allgemeinen Formel $(G)_n$-$OR^{12}$, in der $R^{12}$ einen Alkyl- oder Alkenylrest mit 8 bis 22 C-Atomen, G eine Glykoseeinheit und n eine Zahl zwischen 1 und 10 bedeuten. Bei der Glykosidkomponente $(G)_n$ handelt es sich um Oligo- oder Polymere aus natürlich vorkommenden Aldose- oder Ketose-Monomeren, zu denen insbesondere Glucose, Mannose, Fruktose, Galaktose, Talose, Gulose, Altrose, Allose, Idose, Ribose, Arabinose, Xylose und Lyxose gehören. Die aus derartigen glykosidisch verknüpften Monomeren bestehenden Oligomere werden außer durch die Art der in ihnen enthaltenen Zucker durch deren Anzahl, den sogenannten Oligomerisierungsgrad, charakterisiert. Der Oligomerisierungsgrad n nimmt als analytisch zu ermittelnde Größe im allgemeinen gebrochene Zahlenwerte an; er liegt bei Werten zwischen 1 und 10, bei den vorzugsweise eingesetzten Glykosiden unter einem Wert von 1,5, insbesondere zwischen 1,2 und 1,4. Bevorzugter Monomer-Baustein ist wegen der guten Verfügbarkeit Glucose. Der Alkyl- oder Alkenylteil $R^{12}$ der Glykoside stammt bevorzugt ebenfalls aus leicht zugänglichen Derivaten nachwachsender Rohstoffe, insbesondere aus Fettalkoholen, obwohl auch deren verzweigtkettige Isomere, insbesondere sogenannte Oxoalkohole, zur Herstellung verwendbarer Glykoside eingesetzt werden können. Brauchbar sind demgemäß insbesondere die primären Alkohole mit linearen Octyl-, Decyl-, Dodecyl-, Tetradecyl-, Hexadecyl- oder Octadecylresten sowie deren Gemische. Besonders bevorzugte Alkylglykoside enthalten einen Kokosfettalkylrest, das heißt Mischungen mit im wesentlichen $R^{12}$=Dodecyl und $R^{12}$=Tetradecyl.

Nichtionisches Tensid ist in Mitteln, welche einen erfindungsgemäß verwendeten Wirkstoff enthalten, vorzugsweise in Mengen von 1 Gew.-% bis 30 Gew.-%, insbesondere von 1 Gew.-% bis 25 Gew.-% enthalten.

Als weitere fakultative tensidische Inhaltsstoffe kommen Seifen in Betracht, wobei gesättigte Fettsäureseifen, wie die Salze der Laurinsäure, Myristinsäure, Palmitinsäure oder Stearinsäure, sowie aus natürlichen Fettsäuregemischen, zum Beispiel Kokos-, Palmkern- oder Talgfettsäuren, abgeleitete Seifen geeignet sind. Insbesondere sind solche Seifengemische bevorzugt, die zu 50 Gew.-% bis 100 Gew.-% aus gesättigten $C_{12}$-$C_{18}$-Fettsäureseifen und zu bis 50 Gew.-% aus Ölsäureseife zusammengesetzt sind. Vorzugsweise ist Seife in Mengen von 0,1 Gew.-% bis 10 Gew.-% enthalten.

Gewünschtenfalls können die Mittel auch Betaine und/oder kationische Tenside enthalten, die - falls vorhanden - vorzugsweise in Mengen von 0,5 Gew.-% bis 7 Gew.-% eingesetzt werden.

In einer weiteren Ausführungsform enthält das Mittel wasserlöslichen und/oder gegebenenfalls auch wasserunlöslichen Builder, insbesondere ausgewählt aus Alkalialumosilikat, kristallinem Alkalisilikat mit Modul über 1, monomerem Polycarboxylat, polymerem Polycarboxylat und deren Mischungen, insbesondere in Mengen im Bereich von 0,5 Gew.-% bis 10 Gew.-%.

Das Mittel enthält vorzugsweise 1 Gew.-% bis 10 Gew.-% wasserlöslichen organischen Builder. Zu den wasserlöslichen organischen Buildersubstanzen gehören insbesondere solche aus der Klasse der Polycarbonsäuren, insbesondere Citronensäure und Zuckersäuren, sowie der polymeren Carbonsäuren und Polycarbonsäuren, insbesondere die durch Oxidation von Polysacchariden zugänglichen Polycarboxylate, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative Molekülmasse der Homopolymeren ungesättigter Carbonsäuren liegt im allgemeinen zwischen 5000 g/mol und 200000 g/mol, die der Copolymeren zwischen 2000 g/mol und 200000 g/mol, vorzugsweise 50000 g/mol bis 120000 g/mol, bezogen auf freie Säure. Ein besonders bevorzugtes Acrylsäure-Maleinsäure-Copolymer weist eine relative Molekülmasse von 50000 g/mol bis 100000 g/mol auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei Carbonsäuren und/oder deren Salze sowie als drittes Monomer Vinylalkohol und/oder ein Vinylalkohol-Derivat oder ein Kohlenhydrat enthalten. Das erste saure Monomer oder dessen Salz leitet sich von einer monoethylenisch ungesättigten $C_3$-$C_8$-Carbonsäure und vorzugsweise von einer $C_3$-$C_4$-Monocarbonsäure, insbesondere von (Meth-)acrylsäure ab. Das zweite saure Monomer oder dessen Salz kann ein Derivat einer $C_4$-$C_8$-Dicarbonsäure sein, wobei Maleinsäure besonders bevorzugt ist. Die dritte monomere Einheit wird in diesem Fall von Vinylalkohol und/oder vorzugsweise einem veresterten Vinylalkohol gebildet. Insbesondere sind Vinylalkohol-Derivate bevorzugt, welche einen Ester aus kurzkettigen Carbonsäuren, beispielsweise von $C_1$-$C_4$-Carbonsäuren, mit Vinylalkohol darstellen. Bevorzugte Terpolymere enthalten dabei 60 Gew.-% bis 95 Gew.-%, insbesondere 70 Gew.-% bis 90 Gew.-% (Meth)acrylsäure und/oder (Meth)acrylat, besonders bevorzugt Acrylsäure und/oder Acrylat, und Maleinsäure und/oder Maleinat sowie 5 Gew.-% bis 40 Gew.-%, vorzugsweise 10 Gew.-% bis 30 Gew.-% Vinylalkohol und/oder Vinylacetat. Ganz besonders bevorzugt sind dabei Terpolymere, in denen das Gewichtsverhältnis (Meth)acrylsäure und/oder (Meth)acrylat zu Maleinsäure und/oder Maleat zwischen 1:1 und 4:1, vorzugsweise zwischen 2:1 und 3:1 und insbesondere 2:1 und 2,5:1 liegt. Dabei sind sowohl die Mengen als auch die Gewichtsverhältnisse auf die Säuren bezogen. Das zweite saure Monomer oder dessen Salz kann auch ein Derivat einer Allylsulfonsäure sein, die in 2-Stellung mit einem Alkylrest, vorzugsweise mit einem $C_1$-$C_4$-Alkylrest, oder einem aromatischen Rest, der sich vorzugsweise von Benzol oder Benzol-Derivaten ableitet, substituiert ist. Bevorzugte Terpolymere enthalten dabei 40 Gew.-% bis 60 Gew.-%, insbesondere 45 bis 55 Gew.-% (Meth)acrylsäure und/oder (Meth)acrylat, besonders bevorzugt Acrylsäure und/oder Acrylat, 10 Gew.-% bis 30 Gew.-%, vorzugsweise 15 Gew.-% bis 25 Gew.-% Methallylsulfonsäure und/oder Methallylsulfonat und als drittes Monomer 15 Gew.-% bis 40 Gew.-%, vorzugsweise 20 Gew.-% bis 40 Gew.-% eines Kohlenhydrats. Dieses Kohlenhydrat kann dabei beispielsweise ein Mono-, Di-, Oligo- oder Polysaccharid sein, wobei Mono-, Di- oder Oligosaccharide bevorzugt sind, besonders bevorzugt ist Saccharose. Durch den Einsatz des dritten Monomers werden vermutlich Sollbruchstellen in dem Polymer eingebaut, die für die gute biologische Abbaubarkeit des Polymers verantwortlich sind. Diese Terpolymere weisen im Allgemeinen eine relative Molekülmasse zwischen 1000 g/mol und 200000 g/mol, vorzugsweise zwischen 2000 g/mol und 50000 g/mol und insbesondere zwischen 3000 g/mol und 10000 g/mol auf. Sie können, insbesondere zur Herstellung flüssiger Mittel, in Form wässriger Lösungen, vorzugsweise in Form 30- bis 50-gewichtsprozentiger wässriger Lösungen eingesetzt werden. Alle genannten Polycarbonsäuren werden in der Regel in Form ihrer wasserlöslichen Salze, insbesondere ihre Alkalisalze, eingesetzt.

Als wasserunlösliche, wasserdispergierbare anorganische Buildermaterialien werden insbesondere kristalline oder amorphe Alkalialumosilikate, in Mengen von vorzugsweise nicht über 6 Gew.-%, insbesondere von 0,5 Gew.-% bis 5 Gew.-%, eingesetzt. Unter diesen sind die kristallinen Alumosilikate in Waschmittelqualität, insbesondere Zeolith NaA und gegebenenfalls NaX, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 μm auf und bestehen vorzugsweise zu wenigstens 80 Gew.-% aus Teilchen mit einer Größe unter 10 μm. Ihr Calciumbindevermögen, das nach den Angaben der deutschen Patentschrift DE 24 12 837 bestimmt werden kann, liegt im Bereich von 100 bis 200 mg CaO pro Gramm. Geeignete Substitute oder Teilsubstitute für das genannte Alumosilikat sind kristalline Alkalisilikate, die allein oder im Gemisch mit amorphen Silikaten vorliegen können. Die in den Mitteln als Gerüststoffe brauchbaren Alkalisilikate weisen vorzugsweise ein molares Verhältnis von Alkalioxid zu $SiO_2$ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis $Na_2O:SiO_2$ von 1:2 bis 1:2,8. Derartige amorphe Alkalisilikate sind beispielsweise unter dem Namen Portil® im Handel erhältlich. Solche mit einem

molaren Verhältnis $Na_2O:SiO_2$ von 1:1,9 bis 1:2,8 werden im Rahmen der Herstellung bevorzugt als Feststoff und nicht in Form einer Lösung zugegeben. Als kristalline Silikate, die allein oder im Gemisch mit amorphen Silikaten vorliegen können, werden vorzugsweise kristalline Schichtsilikate der allgemeinen Formel $Na_2Si_xO_{2x+1} \cdot yH_2O$ eingesetzt, in der x, das sogenannte Modul, eine Zahl von 1,9 bis 4 und y eine Zahl von 0 bis 20 ist und bevorzugte Werte für x 2, 3 oder 4 sind. Kristalline Schichtsilikate, die unter diese allgemeine Formel fallen, werden beispielsweise in der europäischen Patentanmeldung EP 0 164 514 beschrieben. Bevorzugte kristalline Schichtsilikate sind solche, bei denen x in der genannten allgemeinen Formel die Werte 2 oder 3 annimmt. Insbesondere sind sowohl β- als auch δ -Natriumdisilikate ($Na_2Si_2O_5 \cdot yH_2O$) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in Mitteln, welche einen erfindungsgemäß zu verwendenden Wirkstoff enthalten, eingesetzt werden. In einer weiteren bevorzugten Ausführungsform der Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5, werden in einer weiteren bevorzugten Ausführungsform von Waschmitteln, welche einen erfindungsgemäß verwendeten Wirkstoff enthalten, eingesetzt.

Zusätzlich können die Mittel weitere in Wasch- oder Reinigungsmitteln übliche Bestandteile enthalten. Zu diesen fakultativen Bestandteilen gehören insbesondere weitere Enzymstabilisatoren, Komplexbildner für Schwermetalle, beispielsweise Aminopolycarbonsäuren, Aminohydroxypolycarbonsäuren, Polyphosphonsäuren und/oder Aminopolyphosphonsäuren, Schauminhibitoren, beispielsweise Organopolysiloxane oder Paraffine, nicht-wässrige Lösungsmittel und optische Aufheller, beispielsweise Stilbendisulfonsäurederivate. Vorzugsweise sind in Mitteln, welche einen erfindungsgemäß verwendeten Wirkstoff enthalten, bis zu 1 Gew.-%, insbesondere 0,01 Gew.-% bis 0,5 Gew.-% optische Aufheller, insbesondere Verbindungen aus der Klasse der substituierten 4,4'-Bis-(2,4,6-triamino-s-triazinyl)-stilben-2,2'-disulfonsäuren, bis zu 5 Gew.-%, insbesondere 0,1 Gew.-% bis 2 Gew.-% Komplexbildner für Schwermetalle, insbesondere Aminoalkylenphosphonsäuren und deren Salze und bis zu 2 Gew.-%, insbesondere 0,1 Gew.-% bis 1 Gew.-% Schauminhibitoren enthalten, wobei sich hier und an sonstiger Stelle der Beschreibung die genannten Gewichtsanteile jeweils auf gesamtes Mittel beziehen.

Lösungsmittel, die zusätzlich zu Wasser eingesetzt werden können, sind vorzugsweise solche, die wassermischbar sind. Zu diesen gehören die niederen Alkohole, beispielsweise Ethanol, Propanol, iso-Propanol, und die isomeren Butanole, Glycerin, niedere Glykole, beispielsweise Ethylen- und Propylenglykol, und die aus den genannten Verbindungsklassen ableitbaren Ether. Mittel mit dem erfindungsgemäß verwendeten polymeren Wirkstoff enthalten vorzugsweise 10 Gew.-% bis 90 Gew.-%, insbesondere 60 Gew.-% bis 80 Gew.-% Wasser.

Zu den gegebenenfalls zusätzlich vorhandenen üblichen Enzymstabilisatoren gehören Aminoalkohole, beispielsweise Mono-, Di-, Triethanol- und -propanolamin und deren Mischungen, niedere Carbonsäuren, Borsäure, Alkaliborate, Borsäure-Carbonsäure-Kombinationen, Borsäureester, Boronsäurederivate, Calciumsalze, beispielsweise Ca-Ameisensäure-Kombination, Magnesiumsalze, und/oder schwefelhaltige Reduktionsmittel.

Zu den geeigneten Schauminhibitoren gehören langkettige Seifen, insbesondere Behenseife, Fettsäureamide, Paraffine, Wachse, Mikrokristallinwachse, Organopolysiloxane und deren Gemische, die darüber hinaus mikrofeine, gegebenenfalls silanierte oder anderweitig hydrophobierte Kieselsäure enthalten können.

Zu den bekanntlich polyesteraktiven schmutzablösevermögenden Polymeren, die zusätzlich zu den erfindungswesentlichen Wirkstoffen eingesetzt werden können, gehören Copolyester aus Dicarbonsäuren, beispielsweise Adipinsäure, Phthalsäure oder Terephthalsäure, Diolen, beispielsweise Ethylenglykol oder Propylenglykol, und Polydiolen, beispielsweise Polyethylenglykol oder Polypropylenglykol. Zu den bevorzugt eingesetzten schmutzablösevermögenden Polyestern gehören solche Verbindungen, die formal durch Veresterung zweier Monomerteile zugänglich sind, wobei das erste Monomer eine Dicarbonsäure HOOC-Ph-COOH und das zweite Monomer ein Diol HO-(CHR[11]-)$_a$OH, das auch als polymeres Diol H-(O-(CHR[11]-)$_a$)$_b$OH vorliegen kann, ist. Darin bedeutet Ph einen o-, m- oder p-Phenylenrest, der 1 bis 4 Substituenten, ausgewählt aus Alkylresten mit 1 bis 22 C-Atomen, Sulfonsäuregruppen, Carboxylgruppen und deren Mischungen, tragen kann, R[11] Wasserstoff, einen Alkylrest mit 1 bis 22 C-Atomen und deren Mischungen, a eine Zahl von 2 bis 6 und b eine Zahl von 1 bis 300. Vorzugsweise liegen in den aus diesen erhältlichen Polyestern sowohl Monomerdioleinheiten -O-(CHR[11]-)$_a$O- als auch Polymerdioleinheiten -(O-(CHR[11]-)$_a$)$_b$O- vor. Das molare Verhältnis von Monomerdioleinheiten zu Polymerdioleinheiten beträgt vorzugsweise 100:1 bis 1:100, insbesondere 10:1 bis 1:10. In den Polymerdioleinheiten liegt der Polymerisationsgrad b vorzugsweise im Bereich von 4 bis 200, insbesondere von 12 bis 140. Das Molekulargewicht oder das mittlere Molekulargewicht oder das Maximum der Molekulargewichtsverteilung bevorzugter schmutzablösevermögender Polyester liegt im Bereich von 250 bis 100 000, insbesondere von 500 bis 50 000. Die dem Rest Ph zugrundeliegende Säure wird vorzugsweise aus Terephthalsäure, Isophthalsäure, Phthalsäure, Trimellithsäure, Mellithsäure, den Isomeren der Sulfophthalsäure, Sulfoisophthalsäure und Sulfoterephthalsäure sowie deren Gemischen ausgewählt. Sofern deren Säuregruppen nicht Teil der Esterbindungen im Polymer sind, liegen sie vorzugsweise in Salzform, insbesondere als Alkali- oder Ammoniumsalz vor. Unter diesen sind die Natrium- und Kaliumsalze besonders bevorzugt. Gewünschtenfalls können statt des Monomers HOOC-Ph-COOH geringe Anteile, insbesondere nicht mehr als 10 Mol-% bezogen auf den Anteil an Ph mit der oben gegebenen Bedeutung, anderer Säuren, die mindestens zwei Carboxylgruppen aufweisen, im schmutzablösevermögenden Polyester enthalten sein. Zu diesen gehören beispielsweise Alkylen- und Alkenylendicarbonsäuren wie Malonsäure, Bernsteinsäure, Fumarsäure, Maleinsäure, Glutarsäure, Adipinsäure, Pimelinsäure, Korksäure, Azelainsäure und Sebacinsäure. Zu den bevorzugten Diolen HO-(CHR$^{11}$-)$_a$OH gehören solche, in denen R$^{11}$ Wasserstoff und a eine Zahl von 2 bis 6 ist, und solche, in denen a den Wert 2 aufweist und R$^{11}$ unter Wasserstoff und den Alkylresten mit 1 bis 10, insbesondere 1 bis 3 C-Atomen ausgewählt wird. Unter den letztgenannten Diolen sind solche der Formel HO-CH$_2$-CHR$^{11}$-OH, in der R$^{11}$ die obengenannte Bedeutung besitzt, besonders bevorzugt. Beispiele für Diolkomponenten sind Ethylenglykol, 1,2-Propylenglykol, 1,3-Propylenglykol, 1,4-Butandiol, 1,5-Pentandiol, 1,6-Hexandiol, 1,8-Octandiol, 1,2-Decandiol, 1,2-Dodecandiol und Neopentylglykol. Besonders bevorzugt unter den polymeren Diolen ist Polyethylenglykol mit einer mittleren Molmasse im Bereich von 1000 g/mol bis 6000 g/mol. Gewünschtenfalls können diese Polyester auch endgruppenverschlossen sein, wobei als Endgruppen Alkylgruppen mit 1 bis 22 C-Atomen und Ester von Monocarbonsäuren in Frage kommen.

Flüssige Mittel werden in der Regel durch einfaches Mischen der Inhaltsstoffe, die in Substanz oder als Lösung in einen automatischen Mischer gegeben werden können, hergestellt.

Beispiele

Beispiel 1: Bestimmung des Aggregationsparameters $X_{ag}$ über die statische Oberflächenspannung

Bei 25 °C wurde unter Verwendung eines TE3-Ring/Plattentensiometers von Lauda die Oberflächenspannung $\gamma$ einer auf pH 8,5 eingestellten wässrigen Lösung von 0,2 g/l linearem Alkylbenzolsufonat (LAS; Disponil® LDBS 55) gemessen. Die Messung wurde mit ansonsten gleichen Lösungen, die aber zusätzlich 0,2 g/l des jeweils zu untersuchenden Polymers enthielten, wiederholt. Der Aggregationsparameter $X_{ag}$ wurde bestimmt, indem der Messwert des Systems ohne Polymer von dem des Systems mit Polymer subtrahiert wurde.

$$X_{ag} = \gamma_1 \, (\text{Tensid} + \text{Polymer}) - \gamma_2 \, (\text{Tensid})$$

Getestet wurden ein N-Vinylpyrrolidon-Homopolymer (PVP), ein N-Vinylcaprolactam-Homopolymer (PVCap), ein Copolymer dieser beiden Monomere (P(VP-co-VCap)) (mittleres Molekulargewicht jeweils 10000 g/mol) sowie ein Vinylpyrrolidon-Vinylsuccinimid-Copolymer (P(VP-co-VSuc); mittleres Molekulargewicht 31000 g/mol) und ein Vinylpyrrolidon-Vinylpiperidon-Copolymer (P(VP-co-VPip); mittleres Molekulargewicht 18000 g/mol). Es wurden die in der folgenden Tabelle 1 angegebenen Aggregationsparameter ermittelt:

Tabelle 1: Aggregationsparameter der Tensid-Polymer Systeme

| System | $X_{ag}$ |
| --- | --- |
| LAS + PVP | 4,7 |
| LAS + PVCap | 6,6 |
| LAS + P(VP-co-VCap) | 6,4 |
| P(VP-co-VSuc) | 5,0 |
| P(VP-co-VPip) | 5,5 |

Die Messung der Oberflächenspannung in Polymeranwesenheit und -abwesenheit wurde auch in einer Alkylbenzolsufonat-haltigen Waschmittelrahmenformulierung durchgeführt. Auch dort war die Wechselwirkung klar erkennbar. Dies belegt, dass die Ausbildung des reinigungsaktiven Aggregats kein auf das isolierte Tensid-Polymer-System beschränktes Phänomen ist. Es handelt sich vielmehr um einen anwendungsrelevanten Effekt der in der Reinigungsanwendung eine verbesserte Leistung bewirkt.

Beispiel 2: Bestimmung des Aggregationsparameters $X_{ag}$ über die dynamische Grenzflächenspannung

Bei 25 °C wurde unter Verwendung eines TVT2-Tropfen-Volumen-Tensiometers von Lauda die dynamische Grenzflächenspannung $\gamma$ einer auf pH 8,5 eingestellten wässrigen Lösung von 0,2 g/l linearem Alkylbenzolsufonat (LAS; Disponil® LDBS 55) gegenüber Isopropylmyristat gemessen. Die Messung wurde mit ansonsten gleichen Lösungen, die aber zusätzlich 0,2 g/l des jeweils zu untersuchenden Polymers enthielten, wiederholt. Die Messungen wurden je nach 1 Minute vorgenommen.

Getestet wurden ein N-Vinylpyrrolidon-Homopolymer (PVP), ein N-Vinylcaprolactam-Homopolymer (PVCap) sowie ein Copolymer dieser beiden Monomere (P(VP-co-VCap)) (mittleres Molekulargewicht jeweils 30000 g/mol). Da die Grenzflächenspannung generell deutlich geringere Werte aufweist als die Oberflächenspannung, wurde der Normierungsfaktor 3 verwendet, um die Werte des aus den Oberflächenspannungsmessungen ermittelten Aggregationsparameters mit den aus den Grenzflächenspannungsmessungen ermittelten Werten vergleichen zu können:

$$X_{ag} = 3 \left[ \gamma_{1Grenzfläche} \left( Tensid + Polymer \right) - \gamma_{2Grenzfläche} \left( Tensid \right) \right]$$

Es wurden die in der folgenden Tabelle 2 angegebenen Aggregationsparameter ermittelt:

Tabelle 2: Aggregationsparameter der Tensid-Polymer Systeme

| System | $X_{ag}$ |
|---|---|
| LAS + PVP | 4,5 |
| LAS + PVCap | 6,0 |
| LAS + P(VP-co-VCap) | 6,0 |

Beispiel 3

Folgende Substanzen wurden auf ihren stabilisierenden Effekt getestet: Ein N-Vinylpyrrolidon-N,N-Dimethylacrylamid-Copolymer (P(VP-co-DMA), mittleres Molekulargewicht 15000 g/mol); ein Vinylpyrrolidon-Vinylpiperidon-Copolymer (P(VP-co-VPip), mittleres Molekulargewicht 18000 g/mol); ein N-Vinylpyrrolidon-Vinylsuccinimid-Copolymer (P(VP-co-VSuc), mittleres Molekulargewicht 31000 g/mol); ein N-Vinylpyrrolidon-N-Vinylcaprolactam-Copolymer (P(VP-co-VCap), mittleres Molekulargewicht 30000 g/mol); und zum Vergleich ein Natrium-Polystyrolsulfonat (PSS) und das Natriumsalz eines sulfonat-terminierten Acrylsäurepolymers (Acusol® 445N).

Dazu wurde eine Protease, umfassend eine Aminosäuresequenz gemäß SEQ ID NO. 2 der internationalen Patentanmeldung WO 2012/080201 A2 (Glu an Position 99 (99E)) in einer Konzentration von 0,04 mg/ml (bezogen auf das aktive Enzym) in einer Lösung von 1% des entsprechenden Polymers und 1 % linearem $C_{10-13}$-Alkylbenzolsulfonat-Na-Salz in 0,1 M Tris-Puffer mit pH 8,0 bei 50°C inkubiert und in regelmäßigen Abständen die Aktivität mittels Messung im AAPF-Test auf Proteaseaktivität überprüft. Die mit der Zeit sinkenden Aktivitäten wurden durch Logarithmierung und Annahme einer Abbaukinetik Pseudo-1.-Ordnung zur Ermittlung der Protease-Halbwertszeiten genutzt. Diese wurden mit der als 1 normierten Halbwertszeit in einer ansonsten gleich zusammengesetzten Lösung ohne Polymer in Relation gesetzt.

Folgende Ergebnisse wurden erzielt:

Tabelle 3: Halbwertszeiten bei Einsatz von Polymer

| Polymer | Halbwertszeit |
|---|---|
| P(VP-co-DMA) | 2,3 |
| P(VP-co-VPip) | 3,0 |
| P(VP-co-VSuc) | 3,8 |
| P(VP-co-VCap) | 4,4 |
| PSS | 1,1 |
| Acusol® 445N | 0,7 |

Die Ergebnisse zeigen, dass die erfindungsgemäß verwendeten Polymere eine Erhöhung der Stabilität auf die 2,3 bis 4,4fache Halbwertszeit bewirken, wohingegen sich mit anderen Polymeren keine Stabilisierung erzielen lässt.

Beispiel 4: Waschversuche

Tabelle 4: Waschmittelzusammensetzungen (Angaben in Gew.-%)

|  | A | B | C | D | E | F | G | H |
|---|---|---|---|---|---|---|---|---|
| $C_{9-13}$-Alkylbenzolsulfonat, Na-Salz | 9 | 10 | 6 | 7 | 5 | 15 | 15 | 9 |
| $C_{12-18}$-Fettalkohol mit 7 EO | 8 | 9 | 6 | 7 | 5 | 6 | 11 | 10 |
| $C_{12-14}$-Fettalkoholsulfat mit 2EO | - | - | 8 | 7 | 10 | 2 | 2 | 5 |
| $C_{12-18}$-Fettsäure, Na-Salz | 4 | 3 | 3 | 3 | 4 | 2 | 4 | 7 |
| Zitronensäure | 2 | 3 | 3 | 2 | 2 | 2 | 2 | 3 |
| Natriumhydroxid, 50 % | 3 | 3 | 2 | 3 | 3 | 3 | 3 | 4 |
| Borsäure | 1 | 1 | 1 | 1 | 1 | 1 | 1 | 1 |
| Purafect Prime® 4000 L | 2 | 4 | 0,7 | 0,2 | 0,7 | 1,2 | 2,2 | 3 |
| Stainzyme® 12.0 L | 1 | 0,5 | 0,25 | 0,125 | 1 | 0,5 | 0,5 | 0,5 |
| Biotouch® NCD | 0,5 | 0,5 | 0,5 | 0,5 | 0,5 | 0,5 | 0,5 | 0,5 |
| Parfüm | 1 | 0,5 | 0,5 | 1 | 1 | 1 | 1 | 1 |
| Propandiol | - | - | - | - | - | 5 | 5 | - |
| Ethanol | 1,5 | 1,5 | 1,5 | 1,5 | 1,5 | 1,5 | 1,5 | 5 |
| PVA/Maleinsäure-Copolymer | 0,1 | - | 0,1 | - | - | - | - | - |
| Optischer Aufheller | - | 0,1 | - | 0,1 | 0,2 | 0,2 | 0,2 | 0,2 |
| Trübungsmittel | 0,2 | - | - | - | - | - | - | - |
| Phosphonsäure, Na-Salz | 0,5 | 0,5 | 0,5 | 0,5 | 0,5 | 0,5 | 0,5 | 0,5 |
| erfindungswesentliches Polymer | 2 | 2 | 2 | 2 | 2 | 2 | 2 | 2 |
| Wasser | auf 100 | | | | | | | |

Haushaltswaschmaschinen (Miele® W 1514) wurden mit 3,5 kg sauberer Begleitwäsche sowie den mit in Tabelle 5 angegebenen standardisierten Anschmutzungen versehenen Testtextilien aus Baumwolle und Schmutzballast beladen. 75 ml des aufgeführten Waschmittels C mit P(VP-co-VCap) mit mittleren Molekulargewicht 30000 g/mol wurden eindosiert und es wurde bei 40°C gewaschen. Nach hängender Trocknung und Mangeln der Testtextilien wurde deren Weißgrad spektralphotometrisch (Minolta® CR200-1) bestimmt. In der nachfolgenden Tabelle 5 sind die Differenzen der Remissionswerte (Rd) zum ansonsten gleich zusammengesetzten Waschmittel ohne erfindungswesentliches Polymer als Mittelwerte aus 5 Bestimmungen sowie die Fehler bei der 5-fach Bestimmung (LSD) angegeben.

Tabelle 5: Waschergebnisse Remissionswertdifferenzen in %

|  | Rd | LSD |
|---|---|---|
| Blaubeersaft | 2,6 | 1,7 |
| Rotwein-1 | 1,8 | 1,3 |
| Salatdressing/Naturschwärze | 2,2 | 1,5 |
| Tee-1 | 2,4 | 1,1 |
| Tee-2 | 1,2 | 0,7 |
| Tee-3 | 1,3 | 0,8 |
| Kaffee | 1,2 | 0,7 |
| Schwarzer-Johannisbeer-Saft | 3,1 | 2,3 |

(fortgesetzt)

| | Rd | LSD |
|---|---|---|
| Brombersaft-1 | 4,9 | 1,3 |
| Brombersaft-2 | 4,5 | 1,8 |
| Rotwein-2 | 2,9 | 1,1 |
| Curry | 1,3 | 0,4 |
| Blut | 3,4 | 2,8 |
| Schokoladenmilch/Ruß | 6,9 | 4 |
| Kakao | 3,1 | 2,4 |
| Schokoladenpudding | 2,7 | 1,4 |
| Schokoladencreme | 3,6 | 2,3 |
| Porridge | 2,5 | 1,9 |
| Heidelbeersaft | 6,3 | 2,4 |
| Mousse au chocolat | 5,1 | 3,7 |
| Kirschsaft | 2,8 | 0,7 |
| Assam-Ceylon-Tee | 2,1 | 0,8 |

Das Waschmittel mit einem erfindungsgemäß zu verwendenden Wirkstoff zeigte eine deutlich bessere Waschleistung als das ansonsten gleich zusammengesetzte Mittel, dem dieser fehlte.

**Patentansprüche**

1. Verwendung von durch Polymerisation von N-Vinylpyrrolidon mit einem Co-Monomeren, ausgewählt aus der Gruppe umfassend N-Vinylcaprolactam, N-Vinylpiperidon, N-Vinylsuccinimid, N-Vinylglutarimid, N-Vinylacetamid, N-Alkyl-N-Vinylacetamid, N-Vinylformamid, N-Alkyl-N-Vinylformamid, Dialkylacrylamid und Mischungen aus mindestens 2 von diesen, erhältlichen Polymeren

   - zur Stabilisierung von Enzymen und/oder
   - zur Verlangsamung des Enzymabbaus und/oder
   - zur Verhinderung des Enzymabbaus

   in flüssigen wasser- und tensidhaltigen Wasch- oder Reinigungsmitteln.

2. Verwendung nach Anspruch 1, **dadurch gekennzeichnet, dass** das Polymer einen Aggregationsparameter $X_{ag}$ mit $X_{ag} > 4$ mN/m, insbesondere $X_{ag}$ im Bereich von 5 mN/m bis 8 mN/m, aufweist.

3. Verwendung nach Anspruch 1 oder 2, **dadurch gekennzeichnet, dass** das Polymer ein mittleres Molekulargewicht in Bereich von 1000 g/mol bis 500000 g/mol, insbesondere von 1100 g/mol bis 150000 g/mol aufweist.

4. Verwendung nach einem der Ansprüche 1 bis 3, **dadurch gekennzeichnet, dass** das Polymer aus neben aus dem Monomer N-Vinylpyrrolidon stammenden Einheiten aus 1 weiterem Monomer stammenden Einheiten zusammengesetzt ist, das die beiden genannten Einheiten im Gewichtsverhältnis von 99:1 bis 1:99, insbesondere von 97:3 bis 70:30, aufweist.

5. Verwendung nach einem der Ansprüche 1 bis 4, **dadurch gekennzeichnet, dass** das Mittel von 0,1 Gew.-% bis 10 Gew.-%, insbesondere von 0,5 Gew.-% bis 2 Gew.-% des Polymers enthält.

6. Verwendung nach einem der Ansprüche 1 bis 5, **dadurch gekennzeichnet, dass** das Enzym ausgewählt wird aus Protease, Amylase, Lipase, Cellulase, Hemicellulase, Mannanase, Pektin-spaltendem Enzym, Tannase, Xylanase, Xanthanase, β-Glucosidase, Carrageenase, Perhydrolase, Oxidase, Oxidoreduktase, sowie deren Gemischen, insbesondere aus Protease, Amylase und Lipase sowie deren Gemischen.

7. Verwendung nach einem der Ansprüche 1 bis 6, **dadurch gekennzeichnet, dass** das Mittel Enzym in einer Menge von 1 x 10⁻⁸ Gew.-% bis 5 Gew.-%, insbesondere von 0,001 Gew.-% bis 3 Gew.-%, an aktivem Protein enthält, wobei auch jedes enthaltene Enzym für sich genommen in den genannten Mengen vorliegen kann, wenn das Mittel mehr als ein Enzym enthält.

8. Verwendung nach einem der Ansprüche 1 bis 7, **dadurch gekennzeichnet, dass** das Mittel von 0,1 Gew.-% bis 20 Gew.-%, insbesondere von 2 Gew.-% bis 15 Gew.-% synthetisches Aniontensid enthält.

9. Verwendung nach Anspruch 8, **dadurch gekennzeichnet, dass** als alleiniges synthetisches Aniontensid oder als Teil der Gesamtmenge an synthetischem Aniontensid Alkali-$C_{10-13}$-alkylbenzolsulfonat, insbesondere solches, in dem die Alkylreste linear sind, eingesetzt wird.

10. Verwendung nach einem der Ansprüche 1 bis 9, **dadurch gekennzeichnet, dass** das Mittel 10 Gew.-% bis 90 Gew.-%, insbesondere 60 Gew.-% bis 80 Gew.-% Wasser enthält.

## Claims

1. The use of polymers that can be obtained by the polymerization of N-vinylpyrrolidone with a co-monomer selected from the group comprising N-vinylcaprolactam, N-vinylpiperidone, N-vinylsuccinimide, N-vinylglutarimide, N-vinylacetamide, N-alkyl-N-vinylacetamide, N-vinylformamide, N-alkyl-N-vinylformamide, dialkylacrylamide and mixtures of at least two of said co-monomers

   - for stabilizing enzymes and/or
   - for slowing down enzyme degradation and/or
   - for preventing enzyme degradation

   in liquid washing or cleaning agents that contain water and surfactants.

2. The use according to claim 1, **characterized in that** the polymer has an aggregation parameter $X_{ag}$ in which $X_{ag}$ > 4 mN/m, $X_{ag}$ being in particular in the range of from 5 mN/m to 8 mN/m.

3. The use according to claim 1 or 2, **characterized in that** the polymer has an average molecular weight in the range of from 1,000 g/mol to 500,000 g/mol, in particular from 1,100 g/mol to 150,000 g/mol.

4. The use according to one of claims 1 to 3, **characterized in that** the polymer is composed of, in addition to units derived from the monomer N-vinylpyrrolidone, units derived from one further monomer, which polymer has the two units mentioned in a weight ratio of from 99:1 to 1:99, in particular from 97:3 to 70:30.

5. The use according to one of claims 1 to 4, **characterized in that** the agent contains from 0.1 wt.% to 10 wt.%, in particular from 0.5 wt.% to 2 wt.%, of the polymer.

6. The use according to one of claims 1 to 5, **characterized in that** the enzyme is selected from protease, amylase, lipase, cellulase, hemicellulase, mannanase, pectin-cleaving enzyme, tannase, xylanase, xanthanase, β-glucosidase, carrageenase, perhydrolase, oxidase, oxidoreductase and mixtures thereof, in particular from protease, amylase and lipase and mixtures thereof.

7. The use according to one of claims 1 to 6, **characterized in that** the agent contains enzyme in an amount of from 1 x 10⁻⁸ wt.% to 5 wt.%, in particular from 0.001 wt.% to 3 wt.%, with respect to the active protein, it also being possible for each enzyme contained to be present individually in the amounts mentioned if the agent contains more than one enzyme.

8. The use according to one of claims 1 to 7, **characterized in that** the agent contains from 0.1 wt.% to 20 wt.%, in particular from 2 wt.% to 15 wt.%, of synthetic anionic surfactant.

**9.** The use according to claim 8, **characterized in that** alkali $C_{10-13}$ alkylbenzene sulfonate, in particular an alkylbenzene sulfonate in which the alkyl groups are linear, is used as the only synthetic anionic surfactant or as part of the total amount of synthetic anionic surfactant.

**10.** The use according to one of claims 1 to 9, **characterized in that** the agent contains from 10 wt.% to 90 wt.%, in particular from 60 wt.% to 80 wt.%, of water.

## Revendications

**1.** Utilisation de polymères pouvant être obtenus par polymérisation de N-vinylpyrrolidone avec un comonomère choisi dans le groupe comprenant le N-vinylcaprolactame, la N-vinylpipéridone, le N-vinylsuccinimide, le N-vinylglutarimide, le N-vinylacétamide, le N-alkyl-N-vinylacétamide, le N-vinylformamide, le N-alkyl-N-vinylformamide, le dialkylacrylamide et des mélanges d'au moins deux de ceux-ci

- pour stabiliser des enzymes et/ou
- pour ralentir la dégradation d'enzymes et/ou
- pour prévenir la dégradation d'enzymes

dans des agents lavants ou nettoyants liquides contenant de l'eau et des tensioactifs.

**2.** Utilisation selon la revendication 1, **caractérisée en ce que** le polymère présente un paramètre d'agrégation $X_{ag}$, $X_{ag}$ étant supérieur à 4 mN/m, en particulier $X_{ag}$ étant compris entre 5 et 8 mN/m.

**3.** Utilisation selon la revendication 1 ou 2, **caractérisée en ce que** le polymère présente un poids moléculaire moyen compris entre 1 000 et 500 000 g/mol, en particulier entre 1 100 et 150 000 g/mol.

**4.** Utilisation selon l'une des revendications 1 à 3, **caractérisée en ce que** le polymère est composé, en plus d'unités dérivées du monomère N-vinylpyrrolidone, d'unités dérivées d'un autre monomère présentant les deux unités mentionnées dans un rapport pondéral de 99:1 à 1:99, en particulier de 97:3 à 70:30.

**5.** Utilisation selon l'une des revendications 1 à 4, **caractérisée en ce que** l'agent contient 0,1 à 10 % en poids, en particulier 0,5 à 2 % en poids du polymère.

**6.** Utilisation selon l'une des revendications 1 à 5, **caractérisée en ce que** l'enzyme est choisie parmi la protéase, l'amylase, la lipase, la cellulase, l'hémicellulase, la mannanase, l'enzyme de division de la pectine, la tannase, la xylanase, la xanthanase, la β-glucosidase, la carraghénase, la perhydrolase, l'oxydase, l'oxydoréductase ainsi que leurs mélanges, en particulier parmi la protéase, l'amylase et la lipase ainsi que leurs mélanges.

**7.** Utilisation selon l'une des revendications 1 à 6, **caractérisée en ce que** l'agent contient une enzyme en une quantité de 1 x $10^{-8}$ à 5 % en poids, en particulier de 0,001 à 3 % en poids, d'une protéine active, chaque enzyme contenue pouvant elle-même également être présente dans les quantités mentionnées si l'agent contient plus d'une enzyme.

**8.** Utilisation selon l'une des revendications 1 ou 7, **caractérisée en ce que** l'agent contient 0,1 à 20 % en poids, en particulier 2 à 15 % en poids d'un tensioactif anionique synthétique.

**9.** Utilisation selon la revendication 8, **caractérisée en ce qu'**un alkylbenzènesulfonate alcalin en $C_{10-13}$, en particulier dans lequel les radicaux alkyles sont linéaires, est utilisé comme unique tensioactif anionique synthétique ou comme partie de la quantité totale de tensioactif anionique synthétique.

**10.** Utilisation selon l'une des revendications 1 à 9, **caractérisée en ce que** l'agent contient 10 à 90 % en poids, en particulier 60 à 80 % en poids d'eau.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2011001173 A1
- WO 9729139 A1
- EP 0181204 A2
- EP 0181205 A2
- US 20020177542 A
- WO 2004014326 A1
- WO 2013034438 A1
- WO 0070006 A
- DE 2412837
- EP 0164514 A
- WO 2012080201 A2

**In der Beschreibung aufgeführte Nicht-Patentliteratur**

- **APIGNY ; JAEGER.** *Biochem. J.,* 1999, vol. 343, 177-183